# EUROPÄISCHE PATENTSCHRIFT

(11) **EP 3 685 781 B1**
(45) Veröffentlichungstag und Bekanntmachung des Hinweises auf die Patenterteilung: **13.04.2022**
(21) Anmeldenummer: 19153586.3
(22) Anmeldetag: 24.01.2019
(51) Int. Cl.: A61B 18/14, A61B 18/00, A61B 18/22

(54) **VORRICHTUNG ZUR GEWEBEKOAGULATION**
DEVICE FOR TISSUE COAGULATION
DISPOSITIF DE COAGULATION DE TISSU

(43) Veröffentlichungstag der Anmeldung: 29.07.2020
(73) Patentinhaber: Erbe Elektromedizin GmbH, 72072 Tübingen (DE); Albert-Ludwigs-Universität Freiburg, 79098 Freiburg (DE)
(72) Erfinder: ATAMAN, Caglar, 79100 Freiburg (DE); VILCHES, Sergio, 79102 Freiburg (DE); ZAPPE, Hans, 8044 Zürich (CH); NEUGEBAUER, Alexander, 72116 Mössingen (DE); FISCHER, Klaus, 72202 Nagold (DE)
(74) Vertreter: Rüger Abel Patentanwälte PartGmbB

(56) Entgegenhaltungen:
- EP-A1- 2 815 695
- WO-A1-2013/164109
- WO-A2-2010/104752
- WO-A2-2011/055368
- DE-A1- 19 860 689
- DE-C2- 19 860 689
- DE-T5- 10 392 791

## Beschreibung

Die Erfindung betrifft eine Einrichtung zur Behandlung von biologischem Gewebe. Insbesondere zur Koagulation und -ablation sind Instrumente bekannt, die mit elektrischer Energie auf das Gewebe einwirken.

Zum Beispiel offenbart die WO 2012/099974 A2 dazu ein Instrument, das elektromagnetische Energie, zum Beispiel in Form von Hochfrequenzstrom und Spannung und einem Argon Plasma zur Koagulation nutzt. Weiter verweist das Dokument auf einen oder mehrere Sensoren, die zum Beispiel zur Erfassung der Leistung der gelieferten Energie der Einwirkungstiefe, der Gewebetemperatur oder anderer physikalischer Parameter, die wie zum Beispiel einer Farbe dienen können. Weiter werden elektromyographische Sensoren zur Erfassung der Elektromyographie der Muscularis Mucosa, ein kalorimetrischer Sensor, ein Serum-Levelsensor und ein bildgebender Sensor genannt.

Die US 2014/0309632 A1 beschreibt eine Einrichtung mit einem Instrument zur Gewebeablation mittels HF-Energie, wobei zur Überwachung des Ablationsfortschritts ein entsprechendes Mess- und Überwachsungssystem vorgesehen ist. Dieses System ist darauf eingerichtet, den Gewebezustand zu erfassen, was durch elektrische Messung am behandelten Gewebe erfolgen kann. Als Messmöglichkeit sind außerdem intravaskulare Ultraschallmessung, optische Kohärenztomographie, optische Kohärenzreflektometrie oder Angiographie genannt.

Die US 5,321,501 beschreibt eine optische Bildgebung an biologischem Gewebe unter Nutzung eines interferenzoptischen Sensors, mit dem sich eine Gewebeoberfläche abtasten lässt. Die Sonde kann ein Endoskop oder Angioskop sein und zum Scannen von Lumen genutzt werden. Zum Parallelscannen werden mehrere optische Pfade bereitgestellt. Zur Vergrößerung des Fokus kann der Fokuspunkt bewegt werden.

Aus der WO 2010/104752 A2 ist ein optisches Multifunktionssondensystem für human- und veterinärmedizinische Anwendungen bekannt. Dieses Sondensystem nutzt als Messverfahren die optische Kohärenztomographie und kann mit dieser eine lineare, flächige oder auch tiefengestufte Abtastung bei einer Gewebeoberfläche vornehmen. Solche Abtastungen werden als A-Scan, B-Scan oder C-Scan bezeichnet. Diese Sonde kann zumindest in einer Ausführungsform auch als HF-Ablationssonde ausgebildet sein.

Außerdem ist aus der US 2007/0213704 A1 ein medizinisches Instrument zur besonders präzisen Ablation von biologischem Gewebe bekannt, wobei zur Ablation zumindest in einer Ausführungsform elektrisch beaufschlagte Elektroden genutzt werden, die Gewebe mittels Funkenbildung abtragen. Anhand der von den aufeinanderfolgenden Funken erzeugten Lichterscheinung kann auf den Gewebetyp geschlossen werden, mit dem der Funke interagiert. Zur Auswertung wird ein spektroskopisches System genutzt, an das das von Funken emittierte Licht mittels einer optischen Faser herangeführt wird. Das Analysesystem ermittelt die Spektren des von den Funken erzeugten Lichts. Durch Vergleich der gewonnenen Spektren mit Referenzdaten kann erkannt werden, wenn Gewebe getroffen wird, das unbeeinflusst bleiben soll, so dass der Ablationsprozess dann sofort abgebrochen werden kann.

Die US 9,060,750 B2 beschreibt ein System mit einem Instrument, das durch Argon-Plasma-Koagulation auf ein Gewebe einwirkt. Mittels optischer Emissionsspektroskopie, bei der das aufgefangene Licht untersucht wird, wird auf das Vorhandensein bestimmter chemischer Substanzen geschlossen.

Die US 7,720,532 B2 beschreibt ein integriertes Instrument, das als vielseitiges Messinstrument, einzusetzen ist. Es enthält einen mehrere Ultraschallwandler umfassenden Ultraschallsensor sowie einen elektrischen Sensor mit einer Zentralelektrode und einer ringförmig, in radialem Abstand darum herum angeordneten Elektrode, die zusammen mit den Ultraschallwandlern an der distalen Stirnseite des Instruments angeordnet sind.

Außerdem ist aus der US 2012/0289954 A1 eine Plasmasonde bekannt, die einen oder mehrere optische Sensoren enthalten kann, die zur Überwachung des Ablationsprozesses vorgesehen sind. Zur Steuerung des Ablationsprozesses können die optischen Sensoren mit Spektrometern verbunden sein, die das aufgefangene Licht analysieren und den Ablationsprozess anhand dessen steuern.

Die DE 103 92 791 T5 offenbart schließlich ein Ablationsinstrument mit einer HF-beaufschlagbaren Ablationselektrode und einem Lichtleiter, der an ein Interferometer angeschlossen ist. Diese Einrichtung wird als Kohärenz-Reflexionsmesser OCR bezeichnet. Mittels des OCR wird z.B zwischen abzutragendem Gewebe und unverletzt zu bleibendem Gewebe unterschieden. In einer abgewandelten Anwendung wird das OCR-Signal verwendet, um die Tiefe eines mittels der Elektrode zu erzeugenden Lochs zu steuern.

Die optische Gewebeerkennung an einem Instrument mit Ablationsinstrument ist auch aus der EP 2 815 695 A1 bekannt.

Davon ausgehend ist es Aufgabe der Erfindung, eine Einrichtung anzugeben, die eine verbesserte Prozesskontrolle ermöglicht.

Diese Aufgabe wird mit einer Einrichtung nach Anspruch 1 gelöst.

Die erfindungsgemäße Einrichtung wird zur Gewebekoagulation eingesetzt. Ein zu der Einrichtung gehöriger Sondenkörper weist mindestens eine Elektrode auf, die mit einer elektrischen Spannung vorzugsweise einer hochfrequenten modulierten oder unmodulierten Spannung U_{HF} beaufschlagbar ist. Von der Elektrode geht dann ein elektrischer Strom aus, der durch ein Plasma und über das zu behandelnde biologische Gewebe fließt. Das Gewebe wird verändert, insbesondere koaguliert und/oder abgetragen.

Dem Sondenkörper ist wenigstens eine Lichtleiteinrichtung zugeordnet, die mit einer Messeinrichtung verbunden ist. Die Messeinrichtung ist als optische Abstandsmesseinrichtung eingerichtet. Die Messeinrichtung ist dabei als interferometrische Abstandsmesseinrichtung ausgebildet, die vorzugsweise mit mehrfarbigem Licht arbeitet und eine absolute Abstandsbestimmung ermöglicht. Als Licht kann kurzkohärentes Licht mit einer Kohärenzlänge, die geringer ist, als der Soll-Abstand der Sonde zu dem Gewebe, insbesondere Weißlicht genutzt werden. Als Licht kann auch langkohärentes Licht mit einer Kohärenzlänge genutzt werden, die größer ist, als der Soll-Abstand der Sonde zu dem Gewebe.

Die Lichtleiteinrichtung weist ein Lichtaufnahmefenster auf, das einen Beobachtungsbereich festlegt. Dieser Beobachtungsbereich überschneidet sich wenigstens teilweise mit dem von dem Sondenkörper ausgehenden Plasmastrahl oder Funken.

Das Lichtaufnahmefenster kann an einer GRIN-Linse oder einem Linsenarray ausgebildet sein, das vorzugsweise mehrere optische Achsen und/oder mehrere Brennpunkte festlegt. Die optischen Achsen schließen paarweise miteinander einen spitzen Winkel, d.h. einen Winkel von höchstens 90°, ein. Sie können zueinander auch parallel orientiert sein. Vorzugsweise ist die GRIN-Linse oder das Linsenarray an eine monofile Lichtleiteinrichtung und diese ihrerseits an eine optische Messeinrichtung angeschlossen.

Dient die optische Messeinrichtung der Abstandsmessung, kann über den Lichtleiter Licht zu den (allen) optischen Achsen und Brennpunkten geliefert werden, und es wird von diesen zurückgestreutes Licht über die Lichtleiteinrichtung zu der Messeinrichtung geliefert. Diese erhält das von den verschiedenen Auftreffpunkten des Lichts der verschiedenen optischen Achsen zurückgestreute Licht und bringt es mit dem Licht der Lichtquelle zur Interferenz. Aus dem gewonnenen Interferenzmuster lassen sich die Abstände des Gewebes zur Sonde an den einzelnen optischen Achsen ermitteln. Auch wenn sich die ermittelten Abstände einzelnen Brennpunkten oder optischen Achsen nicht individuell zuordnen lassen, kann die Messeinrichtung doch darauf eingerichtet sein, wenigstens den kleinsten gemessenen Abstand (Minimalabstand) oder auch eine andere gewünschte Größe, wie beispielsweise den mittleren oder den größten Abstand zu ermitteln.

Die Abstandsmesseinrichtung ist eine interferometrische Abstandsmesseinrichtung. Diese nutzt eine Lichtquelle mit ausreichender Kohärenzlänge, mindestens einen Strahlteiler, einen Lichtempfänger, Lichtleiter und ein Objektiv. Das Objektiv kann eine GRIN-Linse sein; der Strahlteiler kann ein Faserkoppler sein; die Lichtleiter können Lichtleitfasern sein; der Lichtempfänger kann eine Photodiode oder ein Photodiodenarray, z.B. in Gestalt eines Kamerachips sein. Der von den Lichtleitern und dem mindestens einen Strahlteiler festgelegte Lichtweg kann einen Messweg und einen Referenzweg enthalten. Dem Referenzweg und dem Messweg können die gleichen optischen Elemente, insbesondere das vorzugsweise als GRIN-Linse ausgebildete Objektiv sowie der vom Strahlteiler zum Objektiv führenden Teil des Lichtwegs (z.B. des Lichtleiters) angehören. Die dem Gewebe zugewandte Endfläche des Objektivs (z.B. der GRIN-Linse) kann als Referenzspiegel dienen.

Die Einrichtung kann dahingehend ausgelegt werden, dass der Betrieb der Sonde, insbesondere die Aktivierung der Elektrode und eines eventuell von dieser ausgehenden Plasmastrahls, von der Einhaltung bestimmter Abstände, insbesondere von der Nicht-Unterschreitung des Minimalabstands, abhängig gemacht wird. Weil die verschiedenen optischen Achsen der GRIN-Linse oder der Linsenanordnung an verschiedenen Stellen auf ein zu behandelndes Gewebe treffen, kann somit anhand mehrerer Auftreffpunkte sichergestellt werden, dass das biologische Gewebe der Sonde an keiner Stelle zu nahe kommt.

Die erfindungsgemäße Sonde kann insbesondere in einem Chirurgieroboter eingesetzt werden. Die Abstandsmesseinrichtung erleichtert dies erheblich. Der Abstand zwischen der Sonde und dem Gewebe lässt sich anhand der Abstandsmessung sehr viel einfacher einstellen als anhand eines Kamerabildes. Damit ist eine Fernsteuerung der Sonde oder auch eine teilautomatische oder vollautomatische Sondensteuerung möglich.

Die Sonde weist ein oder kann mehrere Elektroden aufweisen. Ebenso kann die Sonde mit einer anderen ähnlich aufgebauten oder baugleichen Sonde zu einer Doppelsonde zusammengefasst sein. Die Lichtleiteinrichtung kann in dem Sondenkörper, an dem Sondenkörper oder auch an einem Halter vorgesehen sein, der zum Beispiel einen oder mehrere Sondenkörper aufnimmt. Nach diesem Prinzip können unterschiedliche Sondenkonfigurationen erstellt werden, die an unterschiedliche Einsatzweisen oder Einsatzorte angepasst sind.

Ist die Messeinrichtung eine interferometrische Abstandsmesseinrichtung, ist die Lichtleiteinrichtung zugleich zum Beleuchten des Messorts, wie auch zur Rückleitung des vom Messort rückgestreuten Lichts zu der Messeinrichtung eingerichtet. Die Messeinrichtung ist dabei vorzugsweise derart ausgebildet, dass sie während Pausen aktiviert wird, in denen von der Elektrode und insbesondere von dem von der Elektrode ausgehenden Funken oder Plasma kein Licht ausgeht. Wird die Elektrode beispielsweise mit gepulster HF-Spannung U_{HF} beaufschlagt, ist die interferometrische Messeinrichtung vorzugsweise in den Pulspausen der HF-Spannung U_{HF} aktiv.

Die Messeinrichtung kann auch zusätzlich als pyrometrische Temperaturmesseinrichtung eingesetzt werden. In diesem Fall ist sie darauf eingerichtet, das von dem behandelten Gewebe ausgehende, insbesondere infrarote Licht aufzunehmen und anhand dessen spektraler Zusammensetzung die Temperatur des Gewebes zu bestimmen. Die Messeinrichtung ist in diesem Fall vorzugsweise wiederum darauf ausgerichtet, in Pulspausen einer gepulsten HF-Spannung U_{HF} aktiviert zu werden, mit der die Elektrode beaufschlagt wird.

Die Messeinrichtung kann auch eine kombinierte Messeinrichtung sein, die sowohl eine interferometrische Abstandsbestimmung, als auch eine pyrometrische Temperaturmessung vornimmt.

Die Messeinrichtung kann zusätzlich dazu eingerichtet sein, den von dem Plasma oder Funken erfassten Gewebetyp mittels optischer Emissionsspektroskopie zu bestimmen. Dazu ist die Messeinrichtung vorzugsweise dazu eingerichtet, während der Pulse der gepulsten Behandlungsspannung (HF-Spannung U_{HF}) Licht aufzunehmen und zu analysieren. Die Analyse des Lichts ist vorzugsweise eine Spektralanalyse, im Rahmen derer das von dem Funken oder Plasma ausgehende Licht einer Spektraluntersuchung unterzogen wird. Zur Gewebeunterscheidung können die gemessenen Spektren mit Referenzspektren bestimmter Gewebetypen verglichen werden. Insbesondere können auch die Spektrallinien von für bestimmte Gewebeschichten typische chemische Elemente als Indikator für Gewebeschichten dienen, z.B. die Spektrallinien von Magnesium oder Calzium. Bei der optische Emissionsspektroskopie ES ist die Intensität des Lichtsignals stark vom Abstand abhängig. Die Berücksichtigung dieses Umstandes bei der Auswertung des Lichtsignals ermöglicht eine wesentliche Verbesserung bei der Auswertung der Emissionsspektren, insbesondere im Hinblick auf ihren Vergleich mit gegebenen Spektren. Ist die optische Messeinrichtung darauf eingerichtet, den gemessenen Abstand mit dem Lichtsignal zu verrechnen, um dieses z.B. auf den Abstand umzurechnen, mit dem das Vergleichsspektrum aufgenommen worden ist, schwindet der störende Einfluss wechselnder Abstände bei der Behandlung. Somit ist es zweckmäßig, wenn die optische Messeinrichtung derart ausgebildet ist, dass sie zur Gewebetypbestimmung sowohl das Spektrum als auch den Abstand heranzieht.

Die Messeinrichtung kann außerdem darauf eingerichtet sein, permanent aktiv zu sein, um in den Pulspausen den Abstand der Sonde von dem Gewebe und/oder die Temperatur des Gewebes zu ermitteln und während der Pulse die Gewebezusammensetzung zu ermitteln.

Mit der Erfindung ist ein nicht erfindungsgemäßes Verfahren zur Gewebeablation durchführbar, bei dem mittels der optischen Messeinrichtung der Ablationsfortschritt und/oder der Abstand des Sondenkörpers zu der Oberfläche eines biologischen Gewebes und/oder der Gewebetyp ermittelt werden. Das Verfahren eignet sich insbesonder zur Mukosaablation. Es können bei der plasmaunterstützten Ablation der Magenschleimhaut von Patienten schichtspezifische Emissionsspektren (ES) erfasst und dem Operateur entsprechend die jeweils behandelten Gewebeschichten angezeigt oder anderweitig signalisiert werden. Das Vordringen des abladierenden Plasmas in die Submucosaschicht kann durch eine Erhöhung mindestens eines ES-Signals von Magnesium im Vergleich zur Mucosaschicht erfasst und angezeigt werden. Das Vordringen des abladierenden Plasmas in die Submucosaschicht kann auch durch eine Erhöhung relativ zur Mucosaschicht mindestens eines ES-Signals von Calcium erfasst und angezeigt werden. Es kann auch ein Zusammentreffen der Erhöhung des ES-Signals von Magnesium mit einer Erhöhung des ES-Signals von Calcium oder eines anderen Markers als Indikator für das Vordringen des Plasmas in die Submucosa genutzt werden.

Insbesondere kann das Vordringen des abladierenden Plasmas in die Muscularis propria (Muskelschicht) durch eine Erhöhung relativ zur Mucosaschicht mindestens eines ES-Signals von Magnesium erfasst und angezeigt werden.

Auch kann das Vordringen des abladierenden Plasmas in die Muscularis propria (Muskelschicht) durch eine Erhöhung relativ zur Mucosaschicht mindestens eines ES-Signals von Calcium oder eines anderen Markers erfasst und angezeigt werden.

Weitere Einzelheiten von Ausführungsformen der Erfindung sind Gegenstand von Unteransprüchen sowie der Zeichnung und der Beschreibung. Es zeigen:
Figur 1 veranschaulicht eine erfindungsgemäße Einrichtung in schematischer Übersichtsdarstellung,
Figur 2 - 4 veranschaulichen unterschiedliche Ausführungsformen von Sonden zur Gewebeablation, in perspektivischer ausschnittsweise Darstellung,
Figur 5 zeigt eine schematische Darstellung des distalen Endes der Lichtleiteinrichtung und einer mit dieser zusammenwirkenden GRIN-Linse anhand deren verschiedenen optischen Achsen,
Figur 6 die GRIN-Linse nach Figur 5 und die von ihr festgelegten Lichtbündel,
Figur 7 eine Interferometereinrichtung in schematischer Darstellung,
Figur 8 ein von dem Interferometer erzeugtes Interferenzspektrum und die sich daraus ergebenden gemessenen Distanzen,
Figur 9 das Zusammenwirken von Komponenten der Einrichtung nach Figur 1, in gesonderter isolierter schematischer Darstellung, und
Figur 10 Diagramme zur Veranschaulichung des Prinzips des Betriebs der Einrichtung nach Figur 1 bzw. 9.

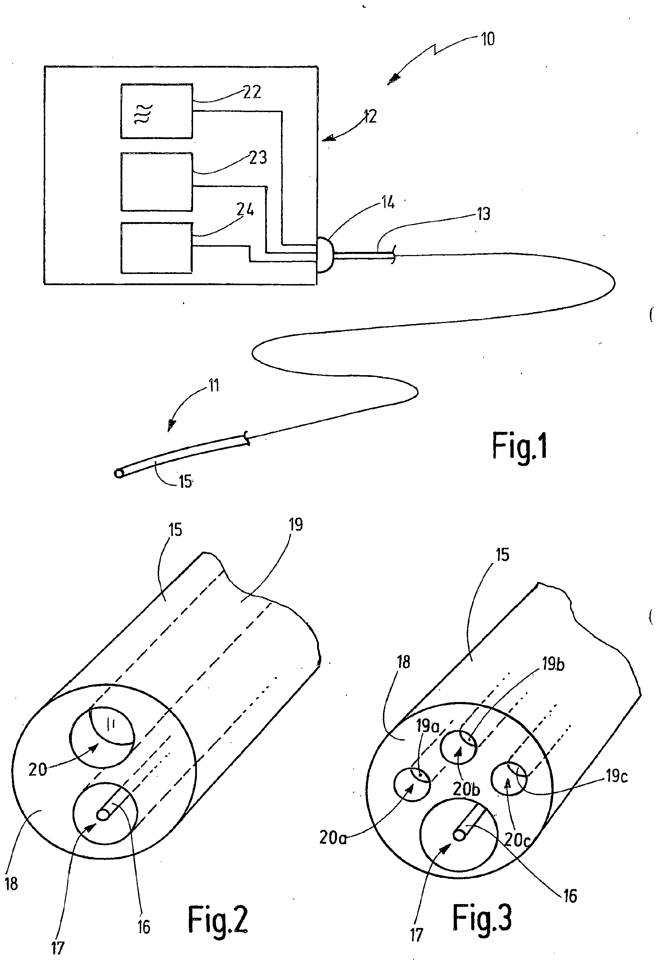

In Figur 1 ist eine Einrichtung 10 veranschaulicht, die zur Gewebekoagulation, zur Gewebeablation oder zur sonstigen Gewebebehandlung dienen kann. Zu der Einrichtung 10 gehören eine Sonde 11 und eine die Sonde 11 speisende Versorgungseinrichtung 12. Diese kann durch ein oder mehrere Geräte gebildet sein und ist in Figur 1 vereinfacht als Block dargestellt. Die nachfolgende Verwendung des Begriffs "Gerät 12" umfasst auch mehrere operativ zusammengefasste Geräte.

Die Sonde 11 kann eine endoskopisch einzusetzende Sonde oder auch ein Instrument für den laparoskopischen Einsatz oder für den offenchirurgischen Einsatz sein. Die nachfolgend erläuterten strukturellen und funktionellen Details gelten, sofern dies nicht prinzipiell ausgeschlossen ist, für jede dieser Bauarten.

Die Sonde 11 ist über eine oder mehrere Leitungen 13 sowie einen oder mehrere Stecker 14 mit dem Gerät 12 verbunden, das die Betriebsleistung und die Medien für den Betrieb der Sonde 11 bereitstellt. Die Sonde 11 weist einen steifen oder flexiblen Sondenkörper 15 auf, in oder an dem eine Elektrode 16 gehalten ist. Die Elektrode 16 ist in einem sich längs durch den Sondenkörper 15 erstreckenden Fluidkanal 17 angeordnet, der zu dem Stecker 14 führt und durch den sich eine die Elektrode 16 mit elektrischer Leistung versorgende elektrische Leitung erstreckt. Der Fluidkanal 17 mündet vorzugsweise an einer Stirnseite 18 des distalen Endes des Sondenkörpers 15. Der Sondenkörper 15 ist außerdem mit einer Lichtleiteinrichtung 19 versehen, die sich von dem distalen Ende des Sondenkörpers 15 bis zu dem Stecker 14 erstreckt. An dem distalen Ende der Lichtleiteinrichtung 19 ist eine Öffnung 20 vorgesehen, durch die Licht ein- und austreten kann und somit von der Lichtleiteinrichtung 19 zu einem Behandlungsort hin abgegeben und von diesem empfangen werden kann. Vorzugsweise verlaufen der Fluidkanal 17 und die Lichtleiteinrichtung 19 in gleicher Richtung durch den Sondenkörper 15.

Wie Figur 3 erkennen lässt, ist es auch möglich, mehrere Lichtleiteinrichtungen 19a, 19b, 19c vorzusehen, die sich durch den Sondenkörper 15 erstrecken und zu dem Stecker 14 führen. Entsprechend sind dann auch an der Stirnfläche 18 mehrere Fenster 20a, 20b, 20c vorgesehen.

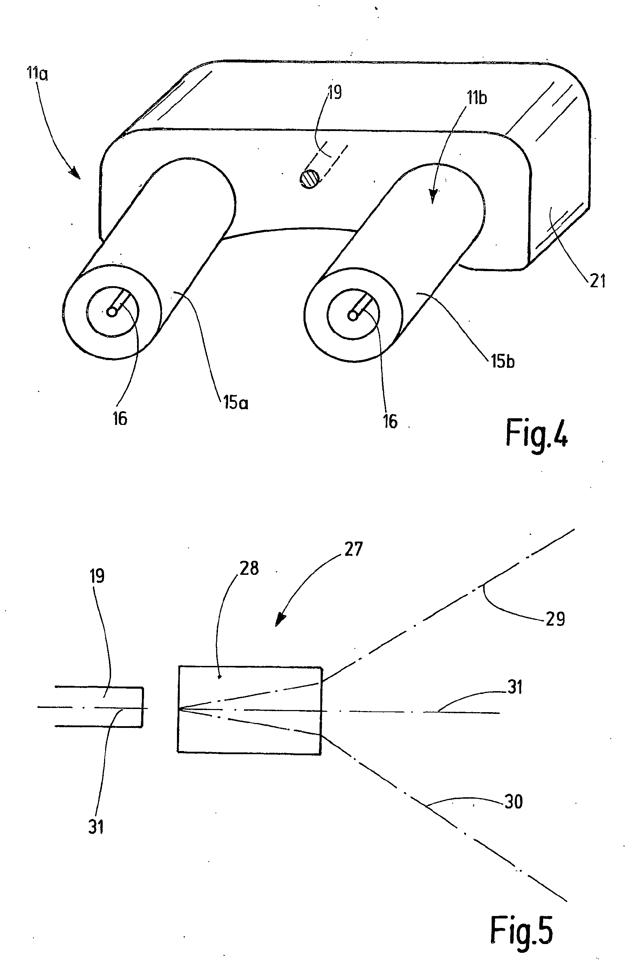

Figur 4 veranschaulicht eine weitere Abwandlung, bei der zwei Sonden 11a, 11b zu einer Zwillingssonde zusammengefasst sind. Die beiden Sonden 11a, 11b können gleich oder unterschiedlich aufgebaut sein. Sie können mit oder ohne optische Lichtleiteinrichtung sowie mit oder ohne Lichtein- oder austrittsfenster ausgebildet sein. In dem in Figur 4 dargestellten Beispiel ist eine Lichtleiteinrichtung 19 an einem Halter 21 angebracht, der die beiden Sonden 11a, 11b miteinander verbindet. Die vorgestellten Sondenvarianten der Figuren 2 bis 4 sind Beispiele, die miteinander kombiniert werden können. Beispielsweise können die Sonden nach Figur 2 und 3 mit einem Halter nach Figur 4 zu einer Zwillingssonde zusammengefasst werden. Auch kann eine der Sonden nach Figur 2 oder 3 mit einer der Sonden 15a oder 15b in dem Halter 21 nach Figur 4 zu einer Zwillingssonde zusammengefasst werden. Allen diesen Anordnungen ist gemeinsam, dass sie wenigstens eine Elektrode 16, wenigstens einen Lichtleiter 19 und wenigstens einen Fluidkanal 17 enthalten. Entsprechend sind in dem Gerät 12 wenigstens ein elektrischer Generator 22, der über den Stecker 14 und die Leitung 13 mit der Elektrode 16 verbunden ist, eine Gasquelle 23, die über den Stecker 14 und die Leitung 13 mit dem Fluidkanal 17 verbunden ist, und eine Messeinrichtung 24 vorgesehen, die über den Stecker 14 und die Leitung 13 mit der Lichtleiteinrichtung 19 verbunden ist.

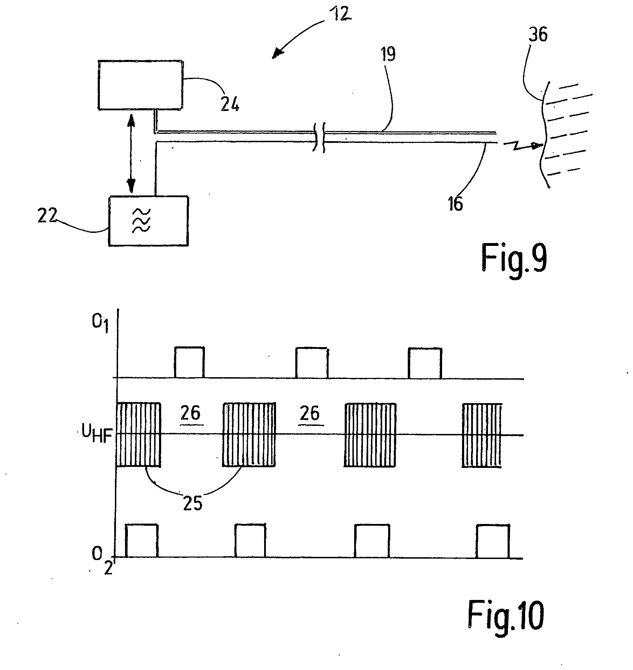

Der Generator 22 ist vorzugsweise ein steuerbarer, von einer nicht weiter dargestellten Steuerschaltung gesteuerter Hochspannungsgenerator. Er ist vorzugsweise darauf eingerichtet, eine hochfrequente Wechselspannung U_{HF} vorzugsweise mit einer Frequenz von deutlich über 100 kHz z.B. 350 kHz abzugeben. Er ist weiter darauf eingerichtet, die hochfrequente Wechselspannung U_{HF} zu modulieren, beispielsweise mit einer Rechteckwelle, so dass eine gepulste Spannungsabgabe mit Pulsen 25 und Pausen 26 gegeben ist, wie es Figur 10 für die hochfrequente Wechselspannung U_{HF} veranschaulicht ist. Die Steuerung bzw. der Generator 22 können darauf eingerichtet sein, das Verhältnis der Zeitdauern der Pulsen 25 und Pausen 26 gemäß vorgegebenen Einstellungen, vorgegebenen Moden oder auch gemäß Steuersignalen zu variieren.

Die Gasquelle 23 kann ebenfalls mit der nicht weiter veranschaulichten Steuereinrichtung verbunden sein, um einen Gasfluss gezielt freizugeben oder zu sperren und/oder um die Flussrate einzustellen. Das Sperren und Freigeben des Gasflusses und/oder das Einstellen der Flussrate kann gemäß Nutzereinstellungen, gemäß gewählten Betriebsmodi und/oder anhand von Steuersignalen erfolgen.

Die Messeinrichtung 24 ist eine optische Messeinrichtung, die als optische Abstandsmesseinrichtung eingerichtet ist und vorzugsweise zusäzlich als pyrometrische Temperaturmesseinrichtung und/oder Messeinrichtung zur Bestimmung des Gewebetyps, vorzugsweise auf Basis der optischen Emissionsspektroskopie, eingerichtet sein kann. Bestimmt die optische Messeinrichtung 24 zugleich mehrere Größen, z.B. Abstand und Temperatur oder Abstand und Gewebetyp, lassen sich bessere Genauigkeiten für die Temperatur oder den Gewebetyp erzielen als ohne Berücksichtigung des Abstandes.

An dem distalen Ende der Lichtleiteinrichtung 19 kann, wie Figur 5 schematisch veranschaulicht, eine als Objektiv dienende Linsenanordnung 27 angeordnet sein, die zum Beispiel eine GRIN-Linse 28 umfasst. Diese kann so ausgestaltet sein, dass sie den Strahlengang in mehrere optische Achsen 29, 30, 31 aufspaltet bzw. zerlegt. Eine mittlere optische Achse 31 kann dabei mit der optischen Achse 31 der Lichtleiteinrichtung 19 identisch sein. Auf einem Kegelmantel um die optische Achse 31 herum können weitere optische Achsen 29, 30 (z.B. 6 an der Zahl) angeordnet sein. Die optischen Achsen 29, 30, 31 können paarweise miteinander einen Winkel, vorzugsweise einen Winkel von höchsten 90°, d.h. einen spitzen Winkel einschließen.

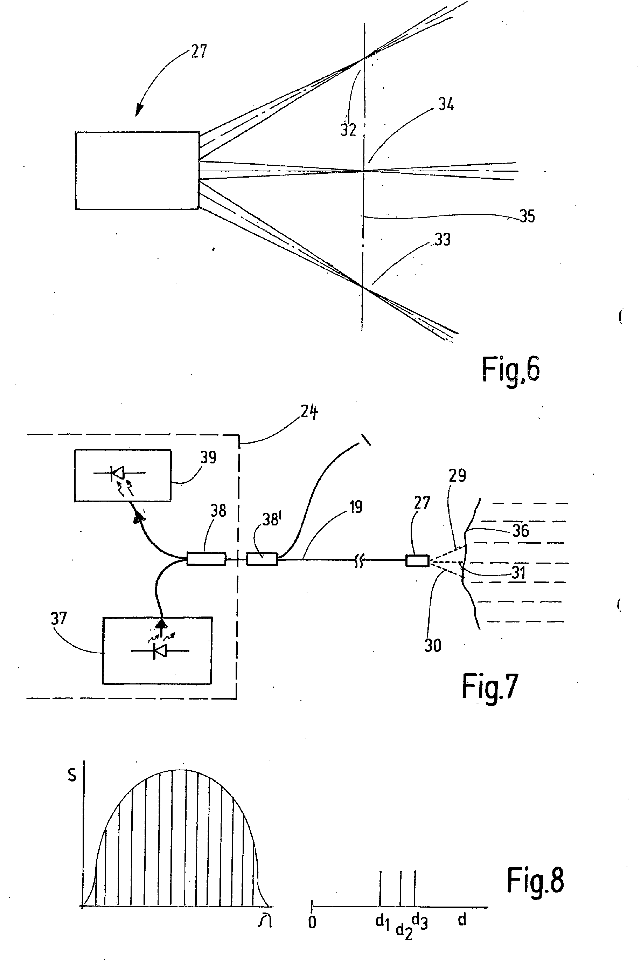

Figur 6 veranschaulicht für die Linsenanordnung 27 nach Figur 5 entsprechend sich ergebenden Lichtbündel, die fokussiert sein können und somit Brennpunkte 32, 33, 34 festlegen. Diese sind vorzugsweise auf einer gemeinsamen Fläche 35, beispielsweise einer Späre, einer Zylinderfläche oder einer Ebene, angeordnet. Außerdem befinden sie sich vorzugsweise in einer Distanz von der GRIN-Linse 28, die ungefähr mit der Distanz übereinstimmt, in der sich die GRIN-Linse 28 beim Einsatz der Sonde 15 von einem biologischem Gewebe 36 entfernt befindet, wie es in Figur 7 schematisch angedeutet ist.

Figur 7 veranschaulicht schematisch und vorwiegend auf die optischen Komponenten beschränkt die Struktur der Messeinrichtung 24, die als interferometrische Messeinrichtung zur Abstandskontrolle oder Abstandsmessung ausgebildet ist. Zu der Messeinrichtung gehört eine Lichtquelle 37, beispielsweise in Gestalt einer Weißlichtquelle oder eines durchstimmbaren Lasers. Dieser ist über einen Faserkoppler 38, der als Strahlteiler dient, an die Lichtleiteinrichtung 19 angeschlossen. Der Faserkoppler 38 ist außerdem mit einem Lichtempfänger 39 verbunden, der sowohl Teile des von der Lichtquelle 37 ausgesandten Lichts wie auch Teile des von der Oberfläche des Gewebes 36 reflektierten Lichts empfängt. Bedarfsweise kann an den Strahlengang über einen weiteren Faserkoppler 38' ein mit einem Reflektor abgeschlossener Referenzlichtweg angekoppelt sein. Bei einer bevorzugten Ausführungsform dient jedoch der Lichtweg in dem Abschnitt der Lichtleiteinrichtung 19 von dem Stahlteiler 38 zu der GRIN-Linse bis zu der Endfläche derselben als Referenzlichtweg. Die Endfläche der GRIN-Linse (oder eines sonstigen Objektivs) reflektiert einen Teil des Lichts und bildet somit den Referenzspiegel. Ein gesonderter Referenzlichtweg kann entfallen.

Je nach Wellenlänge kommt es an dem Lichtempfänger 39 zur konstruktiven oder destruktiven Interferenz, so dass der Lichtempfänger 39 ein Lichtspektrum S empfängt, wie es in Figur 8 links dargestellt ist.

Das Interferometer kann sowohl mit kurzkohärentem Licht (Weißlichtinterferometer) als auch mit Licht größerer Kohärenzlänge arbeitend ausgebildet sein.

Im vorliegenden Ausführungsbeispiel wird zur Abstandsmessung in der Messeinrichtung 24 eine durchstimmbare Lichtquelle 37 eingesetzt, die Licht variabler Wellenlänge abgeben kann. Es werden die einzelnen spektralen Linien des Lichtspektrums beim Durchstimmem der Lichtquelle 37 nacheinander empfangen. Wird hingegen eine Lichtquelle 37 eingesetzt, die mehrere oder alle Farben gleichzeitig emittiert, kann das Spektrum nach Figur 8 durch Spektralzerlegung des von dem Faserkoppler 38 an den Lichtempfänger 39 gelieferten Lichts erzeugt und durch eine entsprechende Vielzahl von Lichtempfangselementen registriert und erzeugt werden.

Das in Figur 8 links dargestellte Spektrum ist durch Interferenz des von der Lichtquelle 37 aus gesandten Lichts mit Licht der verschiedenen Auftreffstellen erzeugt worden, bei denen die optischen Achsen 29, 30, 31 auf die Oberfläche des biologischen Gewebes 36 treffen. Es handelt sich insoweit um ein Summenspektrum. Aus diesem lassen sich die einzelnen Abstandswerte d₁, d₂, d₃ der Schnittstellen der optischen Achsen 29, 30, 31 mit der Oberfläche des Gewebes 36 zu der GRIN-Linse 28 bestimmen. Hinzu kommt, dass weitere nicht veranschaulichte Abstände gemessen werden, die von Reflexionen herrühren, die an tieferen Gewebeschichten auftreten. Dies gilt insbesondere für die Arbeit an lichtdurchlässigen Gewebeschichten.

Die Messeinrichtung 24 kann darauf eingerichtet sein, den kleinsten der Abstandswerte d₁, d₂, d₃ zu ermitteln und diesen der Steuerung des Geräts 12 zur weiteren Verarbeitung bereitzustellen. Die Steuerung kann anhand dieses Werts den Generator 22 steuern, z.B. ein und ausschalten oder in seiner Leistung und/oder in seinem Tastverhältnis (Puls-, Pauseverhältnis) beeinflussen. Auch kann die Steuerung anhand dieses kleinsten Abstandswerts d₁ die Gasquelle 23 ein- oder ausschalten oder zu verstärkter oder verringerter Gasabgabe veranlassen.

Vorzugsweise ist die insoweit beschriebene Interferenzoptisch arbeitende Messeinrichtung 24 während der Pausen 26 der gepulsten, an der Elektrode 16 anliegenden HF-Spannung U_{HF} aktiv, wie es in Figur 10 in dem obersten Diagramm für die erste optische Messung O₁ veranschaulicht ist. Dazu kann der Generator 22, wie es Figur 9 schematisch darstellt, direkt oder durch Vermittlung der Steuerung des Geräts 12 mit der optischen Messeinrichtung 24 so kommunizieren, dass die Messeinrichtung 24 zu dem Generator 22 synchronisiert arbeitet.

Es ist auch möglich, die Messeinrichtung 24 so auszubilden, dass sie zusätzlich oder alternativ eine Gewebeoberflächentemperaturmessung vornimmt, indem sie ebenfalls während der Pausen 26 die von der Oberfläche des Gewebes 36 ausgehende Strahlung, insbesondere Infrarotstrahlung, erfasst und anhand dieser eine pyrometrische Temperaturerfassung durchführt.

Weiter ist es alternativ oder zusätzlich möglich, die optische Messeinrichtung 24 als Teil einer Gewebebestimmungs- oder Klassifikationseinrichtung einzusetzen, die den Typ des von dem Funken oder dem Plasma getroffenen Gewebes durch optische Emissionsspektroskopie bestimmt. Diese Messung ist in Figur 10 im unteren Diagramm O₂ veranschaulicht. Wie ersichtlich, ist die Messeinrichtung 24 zur Durchführung dieser Messung vorzugsweise während der Pulse 25 aktiv. Die Lichtquelle 37 ist dazu inaktiv oder entfällt ganz. Im letzteren Fall kann der Faserkoppler 38 (und, falls vorhanden, auch der Faserkoppler 38') ebenfalls entfallen. Der Lichtempfänger 39 nimmt das von dem Funken oder Plasma ausgehende Licht auf und bestimmt wiederum das Spektrum desselben entsprechend der Darstellung in Figur 8 links. Das erfasste Spektrum kann mit einem Referenzspektrum verglichen werden, um daraus auf den Gewebetyp zu schließen. Zur Bestimmung des Gewebetyps, insbesondere zur Unterscheidung der Mucosa, Submukosa und Muscularis propria (Muskelschicht) bei der Mucosaablation, z.B. zur Reduzierung oder Beseitigung von Ghrelinzellen können auch die typischen Spektrallinien von Magnesium und/oder Calzium erfasst werden, aus deren Vorhandensein und Größe sich ermitteln lasst, mit welcher der Schichten das Plasma interagiert., d.h. welche Schicht abladiert wird.

Die Messeinrichtung 24 kann dementsprechend ein oder mehrere Steuersignale erzeugen, die den geringsten Abstand der Sonde zum Gewebe und/oder die Gewebetemperatur und/oder den Gewebetyp kennzeichnen. Die Steuereinrichtung des Geräts 12 kann darauf eingerichtet sein, den Generator 22 und/oder die Gasquelle 23 entsprechend dieses Signals zu steuern. Beispielsweise kann die Steuereinrichtung den Generator 22 stoppen, sobald ein Mindestabstand der Sonde zu dem Gewebe unterschritten ist. Zugleich oder kurz danach kann die Steuereinrichtung die Gasquelle 23 deaktivieren. Alternativ kann die Sonde vollständig oder zumindest hinsichtlich ihres Abstandes zu dem Gewebe automatisch geführt werden, indem eine Abstandsregeleinrichtung den gewünschten Behandlungsabstand zwischen der Sonde und dem Gewebe anhand der Abstandswessung automatisch einstellt. Es ist auch möglich, den gemessenen Abstand während des Betriebs der Sonde einem Operateur anzuzeigen, damit dieser bei der Führung der Sonde nicht allein auf ein Kamerabild angewiesen ist. Es ist in Kombination mit einer der vorgenannten Varianten oder unabhängig davon auch möglich, die Energiezufuhr, Stromstärke, Spannung, Modulation oder eine sonstige Eigenschaft der von dem Generator gelieferten elektrischen Leistung abstandsabhängig so anzupassen, d.h. dem gemessenen Abstand so nachzuführen, dass sich der Gewebeeffekt durch die Abstandsänderung kaum oder gar nicht ändert.

Zusätzlich oder alternativ kann die Steuereinrichtung den Generator 22 und/oder die Gasquelle 23 deaktivieren sobald die Messeinrichtung 24 anhand des optischen Emissionsspektrums des Plasmas oder des Funkens die Beeinflussung eines Gewebetyps feststellt, der nicht beinflusst werden soll. Das Zusammenwirken der Messeinrichtung 24 mit beispielsweise dem Generator 22 ist in Figur 9 schematisch veranschaulicht. Es kann dabei über das Ein- und Ausschalten des Generators 22 und/oder der Gasquelle 23 hinausgehen. Beispielsweise kann das Puls/Pause-Verhältnis des Generators 22 und/oder die Größe des Gasflusses der Gasquelle 23 anhand des Steuersignals eingestellt oder geregelt werden. Beispielsweise kann das Puls/Pause-Verhältnis der von dem HF-Generator 24 abgegebenen HF-Spannung U_{HF} vermindert werden, wenn die von der Messeinrichtung gemessene Gewebetemperatur einen Grenzwert überschreitet. Zugleich kann der Gasfluss der Gasquelle 23 erhöht oder auch erniedrigt werden. Auf diese Weise lässt sich eine automatische Anpassung des Betriebs des Generators 22 und/oder der Gasquelle 23 an die jeweiligen temporären Betriebszustände der Sonde 11 erreichen.

Bei allen Ausführungsformen der erfindungsgemäßen Einrichtung 10, die zur Gewebekoagulation oder -ablation nutzbar ist, ist an einem Sondenkörper, der zu einem medizinischen Instrument gehören kann, eine hochspannungsbeaufschlagte Elektrode 16 und eine Lichtleiteinrichtung 19 vorgesehen , die an eine Messeinrichtung 24 angeschlossen ist. Diese dient als optische Abstandsmesseinrichtung sowie zumindest optional auch als Einrichtung zur Bestimmung des behandelten Gewebetyps mittels optischer Emissionsspektroskopie ausgebildet sein. Sofern die optische Messeinrichtung als Abstandsmesseinrichtung dient, wird sie als interferenzoptische Messeinrichtung ausgebildet, die darauf eingerichtet ist, den Abstand der Sonde oder der Lichtleiteinrichtung von mehreren Punkten des behandelten Gewebes gleichzeitig zu bestimmen. Dadurch wird es möglich, über einen in einem entsprechenden Gewebebereich, auf den einzelne Messpunkte der optischen Messeinrichtung verteilt sind, den Minimalabstand der Sonde zu erfassen und den Betrieb der Sonde in Abhängigkeit davon zu steuern. Dies kann Spitzenspannung, Leistung, Puls-Pause-Verhältnis oder sonstige elektrische Kenngrößen der verwendeten, an der Elektrode anliegenden HF-Spannung U_{HF}, den Gasfluss oder auch schlicht das Abschalten des HF-Generators und des Gasflusses zur Folge haben.

Die erfindungsgemäße Einrichtung 10 kann zur Gewebekoagulation und/oder zur Gewebeablation eingesetzt werden. Sie umfasst mindestens eine Elektrode 16, die zur Erzeugung eines Funkens oder eines Plasmastrahls dient und dazu an eine elektrische Quelle 20 anschließbar ist. Der Sonde 11 ist eine Messeinrichtung 24 zugeordnet, die Licht in der Nähe der Elektrode 16 aussendet und/oder aufnimmt und den Abstand der Sonde 11 zu dem Gewebe 36 bestimmt. Vorzugsweise wird die Messeinrichtung 24 synchronisiert mit Pulsen oder Pausen der Puls-Pausemodulierten HF-Spannung U_{HF} der Elektrode 16 betrieben, um zeitgleich während des Betriebs des Instruments 11 die gewünschten Messungen vornehmen und den Betrieb des Instruments 11 anhand der gewonnenen Messergebnisse regeln zu können.

### Bezugszeichen:

- 10: Einrichtung zur Gewebeablation oder -Koagulation
- 11: Sonde 11a, 11b
- 12: Speiseeinrichtung / Gerät
- 13: Leitung
- 14: Stecker
- 15: Sondenkörper 15a, 15b
- 16: Elektrode
- 17: Fluidkanal
- 18: Stirnseite
- 19: Lichtleiteinrichtung 19a, 19b, 19c
- 20: Fenster 20a, 20b, 20c
- 21: Halter
- 22: Generator
- 23: Gasquelle
- 24: optische Messeinrichtung
- 25: Puls
- 26: Pause
- 27: Linsenanordnung
- 28: GRIN-Linse
- 29 - 31: optische Achsen
- 32 - 34: Brennpunkte
- 35: Fläche
- 36: biologisches Gewebe
- 37: Lichtquelle
- 38: Faserkoppler
- 39: Lichtempfänger

## Patentansprüche

1. Einrichtung (10), insbesondere zur Gewebekoagulation,
mit einer Sonde (11), die einen Sondenkörper (15) aufweist,
mit mindestens einer Elektrode (16), die mit einer elektrischen Spannung (U_{HF}) beaufschlagbar und in dem Sondenkörper (15) angeordnet ist,
mit mindestens einer Lichtleiteinrichtung (19), die dem Sondenkörper (15) zugeordnet und an eine optische Messeinrichtung (24) anschließbar ist,
wobei die Messeinrichtung (24) als optische, interferometrische Abstandsmesseinrichtung eingerichtet ist, die Licht in der Nähe der Elektrode (16) aussendet und aufnimmt,**dadurch gekennzeichnet,**
**dass** der Sondenkörper (15) wenigstens einen Fluidkanal (17) aufweist, der an eine Gasquelle (23) anschließbar ist,
**dass** die Elektrode (16) in dem Fluidkanal (17) angeordnet ist und
die Messeinrichtung (24) dazu eingerichtet ist, den Abstand der Sonde (11) zu dem Gewebe (36) zu bestimmen.

2. Einrichtung nach Anspruch 1, **dadurch gekennzeichnet, dass** an dem distalen Ende der Lichtleiteinrichtung (19) eine GRIN-Linse (28) oder ein Linsenarray angeordnet ist, das mehrere Brennpunkte (32, 33, 34) festlegt.

3. Einrichtung nach Anspruch 2, **dadurch gekennzeichnet, dass** die Linse (28) mindestens eine, vorzugsweise mehrere optische Achsen (29, 30, 31) festlegend ausgebildet ist, von denen vorzugsweise wenigstens eine parallel oder im spitzen Winkel zu der Elektrode (16) festgelegt ist.

4. Einrichtung nach Anspruch 2, **dadurch gekennzeichnet, dass** die Brennpunkte (32, 33, 34) auf einer vorgegebenen Fläche, vorzugsweise einer Ebene (35) angeordnet sind.

5. Einrichtung nach Anspruch 3 oder 4, **dadurch gekennzeichnet, dass** die Messeinrichtung (24) als Abstandsmesseinrichtung ausgebildet ist und den kleinsten der auf den verschiedenen optischen Achsen (29, 30, 31) bestimmten Abstand (d₁, d₂, d₃) angebend ausgebildet ist.

6. Einrichtung nach einem der vorstehenden Ansprüche, **dadurch gekennzeichnet, dass** die Sonde (15) wenigstens zwei Elektroden (16) aufweist.

7. Einrichtung nach Anspruch 1, **dadurch gekennzeichnet, dass** zur interferometrischen Abstandsmessung eine Lichtquelle (24) vorgesehen ist, die dazu eingerichtet ist, zeitgleich oder zu unterschiedlichen Zeitpunkten Licht unterschiedlicher Wellenlänge (λ) auszusenden.

8. Einrichtung nach einem der vorstehenden Ansprüche, **dadurch gekennzeichnet, dass** die Messeinrichtung (24) zusätzlich als Pyrometer ausgebildet ist, das als Temperaturmesseinrichtung dient.

9. Einrichtung nach einem der vorstehenden Ansprüche, **dadurch gekennzeichnet, dass** die Messeinrichtung (24) zusätzlich ein optisches Emissionsspektrometer ist, das als Gewebetypbestimmungseinrichtung dient.

10. Einrichtung nach einem der vorstehenden Ansprüche, **dadurch gekennzeichnet, dass** die Messeinrichtung (24) eine Kombination aus wenigstens einer Photodiode mit wenigstens einem optischen Filter ist.

11. Einrichtung nach einem der vorstehenden Ansprüche, bei der der Sondenkörper (15),
die mindestens eine Elektrode (16) und
die mindestens eine Lichtleiteinrichtung (19) als Teil eines Instruments (15) zur Gewebebehandlung und
die Messeinrichtung (24) als Teil eines das Instrument speisenden Geräts (12) ausgebildet sind.

12. Einrichtung nach Anspruch 11, **dadurch gekennzeichnet, dass** das Gerät (12) einen Generator (22) zur Erzeugung einer hochfrequenten gepulsten Spannung (U_{HF}) mit Pulsen (25) und Pulspausen (26), an den die Elektrode (16) des Instruments (15) anschließbar ist, und eine optische Messeinrichtung (24) umfasst, an die die Lichtleiteinrichtung (19) des Instruments (15) anschließbar ist,
wobei die optische Messeinrichtung (24) wenigstens während der Pulspausen (26) aktiv ist.

## Claims

1. Device (10), in particular for tissue coagulation,
with a probe (11) having a probe body (15),
with at least one electrode (16) which can be loaded with an electrical voltage (U_{HF}) and is arranged in the probe body (15),
with at least one light-guide device (19) which is assigned to the probe body (15) and can be connected to an optical measuring device (24),
wherein the measuring device (24) is configured as an optical, interferometric distance measuring device which emits and receives light in the vicinity of the electrode,
**characterised in that**
the probe body (15) has at least one fluid channel (17) which can be connected to a gas source (23),
the electrode (16) is arranged in the fluid channel (17), and
the measuring device (24) is configured to determine the distance of the probe (11) from the tissue (36).

2. Device according to claim 1, **characterised in that** a GRIN lens (28) or a lens array, which establishes several focal points (32, 33, 34), is arranged at the distal end of the light-guide device (19).

3. Device according to claim 2, **characterised in that** the lens (28) is configured to establish at least one, preferably several optical axes (29, 30, 31), of which preferably at least one is established parallel to or at an acute angle to the electrode (16).

4. Device according to claim 2, **characterised in that** the focal points (32, 33, 34) are arranged on a predefined surface, preferably a plane (35).

5. Device according to claim 3 or 4, **characterised in that** the measuring device (24) is configured as a distance measuring device, and is configured to show the smallest of the distances (d₁, d₂, d₃) determined on the various optical axes (29, 30, 31).

6. Device according to any of the preceding claims, **characterised in that** the probe (15) has at least two electrodes (16).

7. Device according to claim 1, **characterised in that** for interferometric distance measurement, a light source (24) is provided which is configured to emit light of different wavelengths (λ) at the same time or at different times.

8. Device according to any of the preceding claims, **characterised in that** the measuring device (24) is additionally configured as a pyrometer which serves as a temperature measuring device.

9. Device according to any of the preceding claims, **characterised in that** the measuring device (24) is also an optical emission spectrometer which serves as a tissue-type determination device.

10. Device according to any of the preceding claims, **characterised in that** the measuring device (24) is a combination of at least one photodiode with at least one optical filter.

11. Device according to any of the preceding claims, wherein:
- the probe body (15),
- the at least one electrode (16), and
- the at least one light-guide device (19)
are configured as part of an instrument (15) for tissue treatment, and
the measuring device (24) is configured as part of a device (12) supplying the instrument.

12. Device according to claim 11, **characterised in that** the device (12) comprises a generator (22) for generating a high-frequency pulsed voltage (U_{HF}) with pulses (25) and pulse pauses (26), to which the electrode (16) of the instrument (15) can be connected, and an optical measuring device (24) to which the light-guide device (19) of the instrument (15) can be connected,
wherein the optical measuring device (24) is active at least during the pulse pauses (26).

## Revendications

1. Dispositif (10), destiné en particulier à la coagulation de tissu,
comprenant une sonde (11) qui présente un corps de sonde (15), comprenant au moins une électrode (16) à laquelle peut être appliquée une tension électrique (U_{HF}) et qui est disposée dans le corps de sonde (15),
comprenant au moins un dispositif de guidage de lumière (19) qui est associé au corps de sonde (15) et peut être raccordé à un dispositif de mesure optique (24),
le dispositif de mesure optique (24) étant conçu comme dispositif de mesure de distance optique interférométrique, qui émet et reçoit de la lumière à proximité de l'électrode (16), **caractérisé**
**en ce que** le corps de sonde (15) présente au moins un canal à fluide (17) qui peut être raccordé à une source de gaz (23),
**en ce que** l'électrode (16) est disposée dans le canal à fluide (17), et
le dispositif de mesure (24) est conçu pour déterminer la distance de la sonde (11) par rapport au tissu (36).

2. Dispositif selon la revendication 1, **caractérisé en ce que**, à l'extrémité distale du dispositif de guidage de lumière (19), il est prévu une lentille GRIN (28) ou un réseau de lentilles qui définit plusieurs foyers (32, 33, 34).

3. Dispositif selon la revendication 2, **caractérisé en ce que** la lentille (28) est réalisée de manière à définir au moins un, de préférence plusieurs axes optiques (29, 30, 31), parmi lesquels de préférence au moins un est défini parallèlement ou à angle aigu par rapport à l'électrode (16).

4. Dispositif selon la revendication 2, **caractérisé en ce que** les foyers (32, 33, 34) sont placés sur une surface prédéterminée, de préférence un plan (35).

5. Dispositif selon la revendication 3 ou 4, **caractérisé en ce que** le dispositif de mesure (24) est réalisé comme dispositif de mesure de distance et est conçu de manière à indiquer la plus petite distance (d₁, d₂, d₃) déterminée sur les différents axes optiques (29, 30, 31).

6. Dispositif selon l'une des revendications précédentes, **caractérisé en ce que** la sonde (15) présente au moins deux électrodes (16).

7. Dispositif selon la revendication 1, **caractérisé en ce que**, pour la mesure de distance interférométrique, il est prévu une source de lumière (24) qui est conçue pour émettre de la lumière de longueurs d'ondes (λ) différentes, simultanément ou à des instants différents.

8. Dispositif selon l'une des revendications précédentes, **caractérisé en ce que** le dispositif de mesure (24) est en outre réalisé comme pyromètre qui sert de dispositif de mesure de température.

9. Dispositif selon l'une des revendications précédentes, **caractérisé en ce que** le dispositif de mesure (24) est en outre réalisé comme spectromètre à émission optique qui sert de dispositif de détermination de tissu.

10. Dispositif selon l'une des revendications précédentes, **caractérisé en ce que** le dispositif de mesure (24) est une combinaison d'au moins une photodiode munie d'au moins un filtre optique.

11. Dispositif selon l'une des revendications précédentes, dans lequel
le corps de sonde (15),
l'électrode (16), au nombre d'au moins une, et
le dispositif de guidage de lumière (19), au nombre d'au moins un, sont réalisés en tant que partie d'un instrument (15) destiné au traitement de tissu, et
le dispositif de mesure (24) est réalisé en tant que partie d'un appareil (12) alimentant l'instrument.

12. Dispositif selon la revendication 11, **caractérisé en ce que** l'appareil (12) comprend un générateur (22) pour la production d'une tension pulsée à haute fréquence (U_{HF}) comportant des impulsions (25) et des intervalles entre impulsions (26), auquel peut être raccordée l'électrode (16) de l'instrument (15), et un dispositif de mesure optique (24) auquel peut être raccordé le dispositif de guidage de lumière (19) de l'instrument (15),
le dispositif de mesure optique (24) étant actif au moins pendant les intervalles entre impulsions (26).
